# EUROPEAN PATENT APPLICATION

(11) **EP 3 750 988 A1**
(43) Date of publication of application: **16.12.2020**
(21) Application number: 19751544.8
(22) Date of filing: 07.02.2019
(51) Int. Cl.: C12N 5/078, A61K 35/17, A61P 31/04, A61P 31/12, A61P 31/14, A61P 31/16, A61P 31/18, A61P 31/20, A61P 35/00, A61P 35/02, C12N 1/00, C12N 5/0783

(54) **IMPROVED ALPHA BETA T PROCESSED CELL PRODUCTION METHOD**

(30) Priority: 09.02.2018 JP 2018022488; 28.09.2018 JP 2018184167
(71) Applicant: Osaka University, Suita-shi, Osaka 565-0871 (JP); Medinet Co., Ltd., Shinagawa-ku Tokyo 140-0012 (JP)
(72) Inventor: TOYOFUKU, Toshihiko, Suita-shi, Osaka 565-0871 (JP); SASAWATARI, Shigemi, Tokyo 140-0012 (JP)
(74) Representative: J A Kemp LLP
(86) International application number: PCT/JP2019/004357
(87) International publication number: WO 2019/156145

(57) **Abstract**

Provided are a production method for cytotoxic T cells with increased recognition of cancer cells, a cancer treatment drug including said cytotoxic T cells, and a treatment method using said treatment drug.

## Description

### TECHNICAL FIELD

The present invention relates to a production method for cytotoxic T cells with enhanced cytotoxicity against cancer cells, a cancer treatment drug comprising said cytotoxic T cells, and a treatment method using said cancer treatment drug.

### BACKGROUND ART

Immune cell therapy (adoptive T cell therapy) is one of cancer immunotherapies that aim to treat cancer cells in the body by the immune response by expanding T cells collected from a patient in vitro and returning the cells to the body of the patient. Depending on the type of the cells to be used, the immune cell therapy is mainly classified as follows:
1) dendritic cell vaccine therapy; 2) αβ T cell therapy; 3) cytotoxic T lymphocyte (CTL) therapy; 4) γδ T cell therapy; 5) natural killer (NK) cell therapy; and the like, and these therapies have been actively performed (NON-PATENT LITERATURE 1).

The dendritic cell vaccine therapy refers to a method in which the cancer cells to be targeted are phagocytosed by dendritic cells (DCs) differentiated from monocytes in peripheral blood, the resultant cells are returned to the body, and the cells function as "antigen presenting cells (APCs)" that play a role of transmitting markers (antigens) of cancer or a pathogen to cells (mainly T cells) having an ability to damage the cancer cells or to B cells having an antibody production function, and thus the cancer is treated.

The αβ T cell therapy refers to a treatment method in which lymphocytes contained in peripheral blood are cultured for around 2 weeks by using interleukin-2 (IL-2) and anti-CD3 antibodies, and the whole cells is activated and proliferated, and then returned to the body. Most of the T lymphocytes in peripheral blood are αβ T cells having αβ-type T cell receptors (TCRs), and most of the proliferated cells are also αβ T cells.

The cytotoxic T lymphocyte (CTL) therapy is a treatment method which generally performed with T cells stimulated by DCs that have incorporated cancer cells or cancer antigens outside the body, which lead to expand T cells having cancer antigen-specific cytotoxicity, and then thus expanded cells are returned to the body. However, there are only a few cancer-specific CTLs in blood, and it is difficult to obtain a sufficient number of CTLs for the treatment.

The γδ T cells are major cells responsible for the biological defense response called lymphocyte stress (cell injury) monitor mechanism (lymphoid stress-surveillance). The γδ T cell therapy refers to a treatment method in which γδ T cells having γδ-type T cell receptors (Vγ9Vδ2 receptors) that are present in only a few numbers (1 to 5%) in the peripheral blood collected from a patient are selectively proliferated, and thus proliferated cells are returned to the body. With the recognition, by the γδ T cells, of a molecule or the like that is relatively commonly expressed on a surface of a cancer cell, which is different from "antigen" recognized by an αβ T cell, the γδ T cells acquire an "antigen non-specific" anti-tumor effect for killing cancer (PATENT LITERATURE 1 and NON-PATENT LITERATURE 2).

The NK (natural killer) cell therapy refers to a method in which cells that have high ability to damage abnormal cells, such as NK cells contained in peripheral blood are activated and proliferated by using multiple stimulants such as IL-2, and thus activated and proliferated cells are returned to the body to treat cancer. The NK cell is a kind of lymphocytes that are responsible for innate immune system. The NK cell has strong cytotoxic ability, and is, in particular, an important cell for elimination of virus-infected cells because the NK cell has a mechanism of recognizing and killing a cell in which the expression of MHC class I molecule is decreased or disappeared, and functions as a main effector cell in antibody-dependent cell-mediated cytotoxicity (ADCC).

The immune cell therapy has an advantage of almost no side effects, as compared with the three conventional major therapies of surgery, radiotherapy, and chemotherapy, and is a treatment method that is actually performed as advanced medical care for a patient suffering from lung cancer as the fourth cancer treatment method. However, in order to further improve the therapeutic effect on cancer, immune cells, which: 1) enhance the cytotoxic function and can maintain such function in the body for a long period of time; 2) have high cancer specificity; and 3) do not cause functional deterioration even in the cancer tissue, are required.

### CITATION LIST

### PATENT LITERATURE

PATENT LITERATURE 1: JP-B-3056230
PATENT LITERATURE 2: WO 2013/153800

NON-PATENT LITERATURE 1: HUMAN CELL, Vol. 5, No. 3 (1992)
NON-PATENT LITERATURE 2: Blomed & Pharmacother, (1993) 47, 73-78
NON-PATENT LITERATURE 3: BIOTHRAPY, Vol. 4, No. 10, (1990)
NON-PATENT LITERATURE 4: J Immunol. 2012 Feb 15; 188 (4): 1847-55
NON-PATENT LITERATURE 5: Curr Immunol Rev. 2009 Feb; 5 (1): 22-34
NON-PATENT LITERATURE 6: BLOOD, 15 2004, Vol. 103 (8), 3065-3072

### SUMMARY OF INVENTION

### TECHNICAL PROBLEM

Provided herein is an immune cell therapy that is more aggressive against cancer, focusing on the αβ T cell therapy that has been conventionally performed, among the immune cell therapies. Furthermore, provided herein is an immune cell therapy that is effective also against a disorder causing other cellular stress (for example, viral infection, bacterial infection, drug stress, oxidative stress, etc.) (NON-PATENT LITERATURE 5).

### SOLUTION TO PROBLEM

As described above, the αβ T cell is presented with a cancer antigen by a dendritic cell or the like, and attacks a cancer cell presenting the antigen as a marker. However, in addition to that, it is known that there is a mechanism having antigen non-specific cancer-cytotoxic activity (NON-PATENT LITERATURE 6).

As a result of keen study, the present inventors have found that in in-vitro culturing αβ T cells, when a compound [2-deoxy-d-glucose (hereinafter, referred to as "2DG")] that alters glycosylation (N-linked glycan) of protein through mannose metabolism is added into a cell culture solution, the prepared immune cells acquire a strong antigen non-specific anti-tumor effect (effector function) as compared with the conventional αβ T processed cells.

### ADVANTAGEOUS EFFECTS OF INVENTION

Conventionally, 2DG was administered sometimes as it is as a medicine. However, the 2DG simultaneously acts also on a cancer cell and inhibits cell surface expression of a NKG2D ligand that is a target molecule of an immune cell, and therefore, there is a possibility that cancer cells escape from the recognition of NK cells or T cells. (NON-PATENT LITERATURE 4). To the contrary, the invention of the present application can provide cancer immunotherapy specifically and selectively to the cancer expressing a specific NKG2D ligand by using 2DG during in-vitro culture of immune cells. Furthermore, in a case where the 2DG is administered to the body, by altering the glycosylation on a cell surface of the αβ T cell, it can be expected to avoid the suppression of effector function by cancer cells.

### BRIEF DESCRIPTION OF DRAWINGS

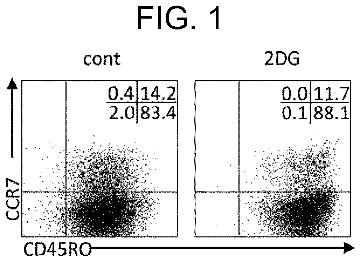

FIG. 1 shows fluorescence-activated cell sorting (FACS) plots indicating the distribution of CD8⁺ T-cell subsets. Each of the numbers in the drawing indicates the percentage of cells in each quadrant. cont: anti-CD3 antibody stimulation, cultured in a medium containing IL-2; 2DG: anti-CD3 antibody stimulation, cultured in a medium containing IL-2 and 2DG.

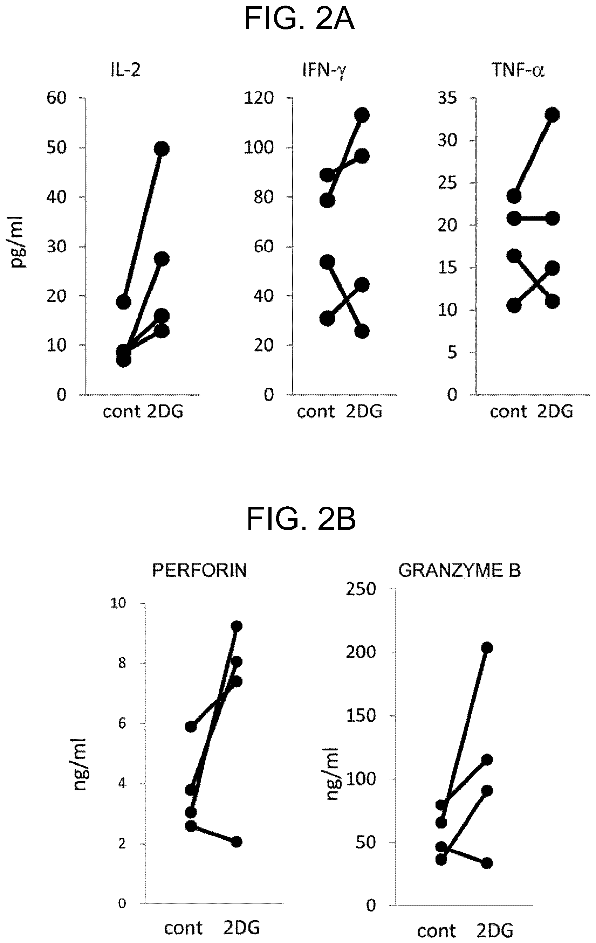

FIG. 2A shows the amount of cytokines in a culture supernatant after 24 hours from the replacement of αβ T processed cells cultured in the absence of 2DG (cont) or in the presence of 2DG (2DG) in a RPMI 1640 + 10% FCS medium. A line was drawn for each donor from which the samples were derived. The amount was measured by Bio-plex. cont: anti-CD3 antibody stimulation, cultured in a medium containing IL-2; 2DG: anti-CD3 antibody stimulation, cultured in a medium containing IL-2 and 2DG.
FIG. 2B shows the amount of each of perforin and granzyme in a culture supernatant after 24 hours from the phorbol myristate acetate (PMA)/ionomycin stimulation after the replacement of the αβ T processed cells cultured in the absence of 2DG (cont) or in the presence of 2DG (2DG) in a RPMI 1640 + 10% FCS medium. The amount was measured by an ELISA assay.

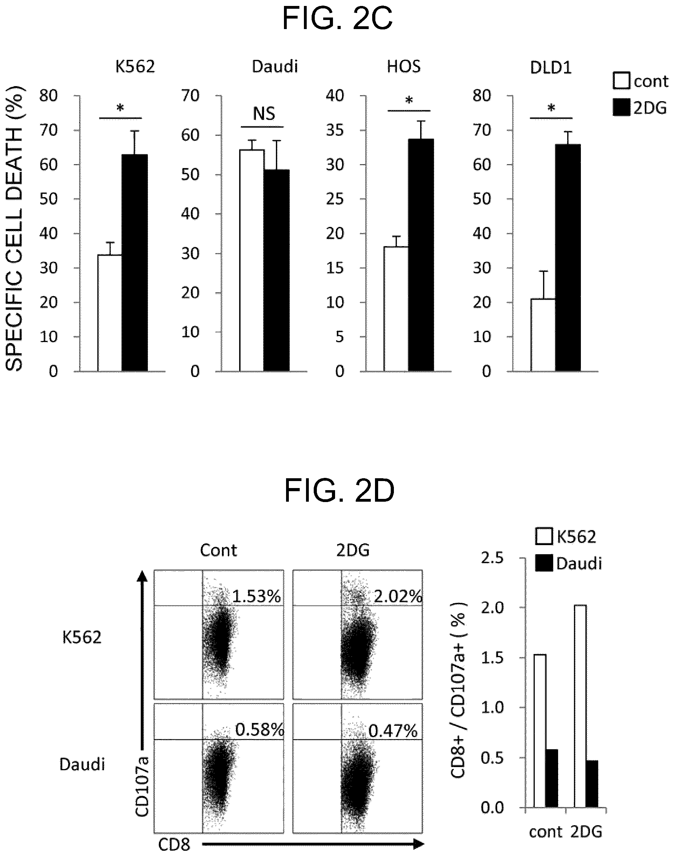

FIG. 2C shows the cytotoxic activity of the αβ T processed cells against cancer cells, which were cultured in the absence of 2DG (cont) or in the presence of 2DG (2DG). Cytotoxic activity was calculated from the fluorescence attenuation of the damaged cancer cells, the maximum fluorescence attenuation of the cancer cells treated with 1% NP40, and the natural attenuation of only the cancer cells, after co-culturing the fluorescent-labeled cancer cells and the αβ T processed cells. NS: no significant difference; and * P < 0.01.
FIG. 2D shows the percentage of CD8⁺ T cells having degranulation marker CD107a in a case where the αβ T processed cells are co-cultured with cancer cells. The αβ T processed cells were cultured in the absence of 2DG (cont) or in the presence of 2DG (2DG).

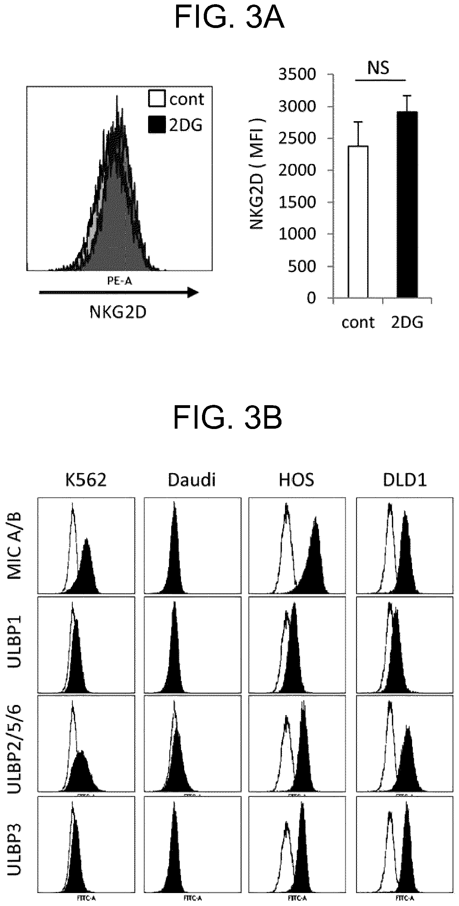

FIG. 3A shows the expression level of NKG2D of the αβ T processed cells cultured in the absence of 2DG (cont) or in the presence of 2DG (2DG). NS: no significant difference.
FIG. 3B shows the expression of NKG2D ligands on surfaces of cancer cells.

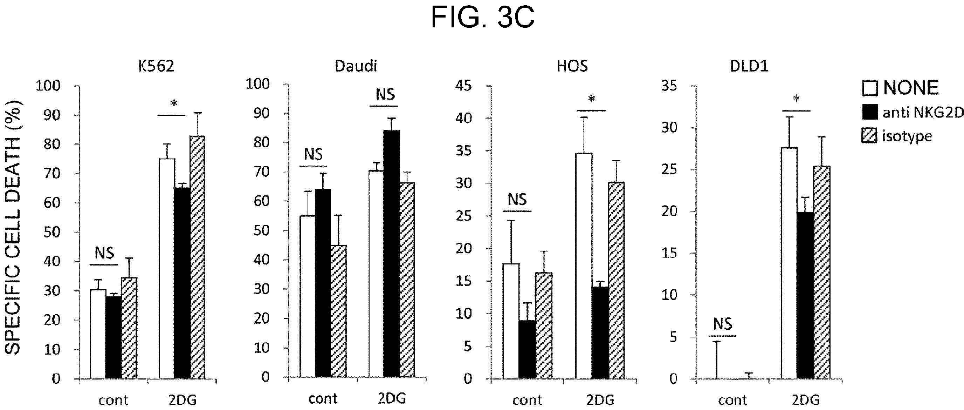

FIG. 3C shows the suppression of cytotoxic activity by an anti-NKG2D antibody. The αβ T processed cells were cultured in the absence of 2DG (cont) or in the presence of 2DG (2DG). NS: no significant difference; and * P < 0.01.

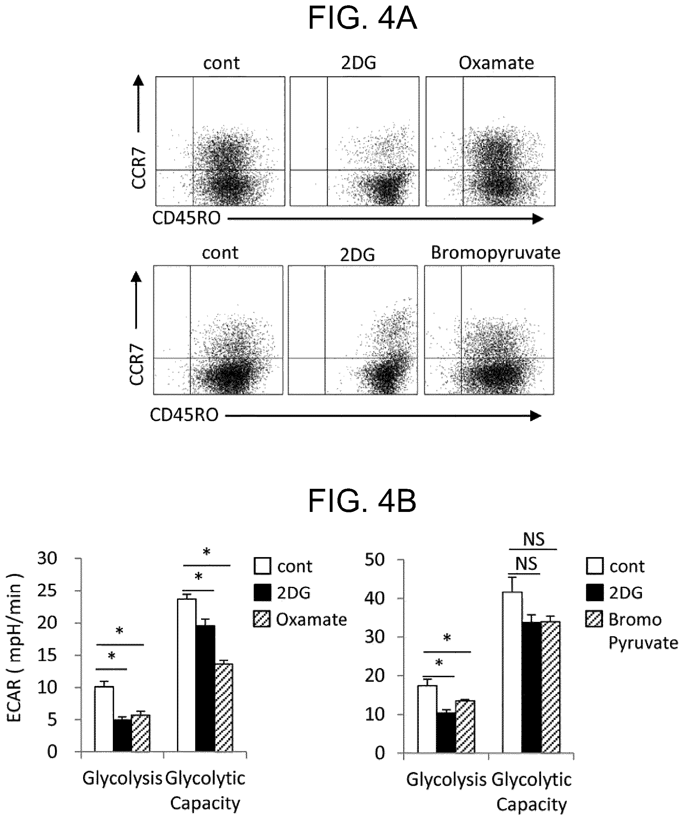

FIG. 4A shows FACS plots indicating the distribution of CD8⁺ T cell subsets of the αβ T processed cells cultured with a glycolytic inhibitor. Each of the numbers in the drawing indicates the percentage of cells in each quadrant.
FIG. 4B shows the influence of 2DG, oxamate or bromo pyruvate on the glycolysis system. NS: no significant difference; and * P < 0.01.

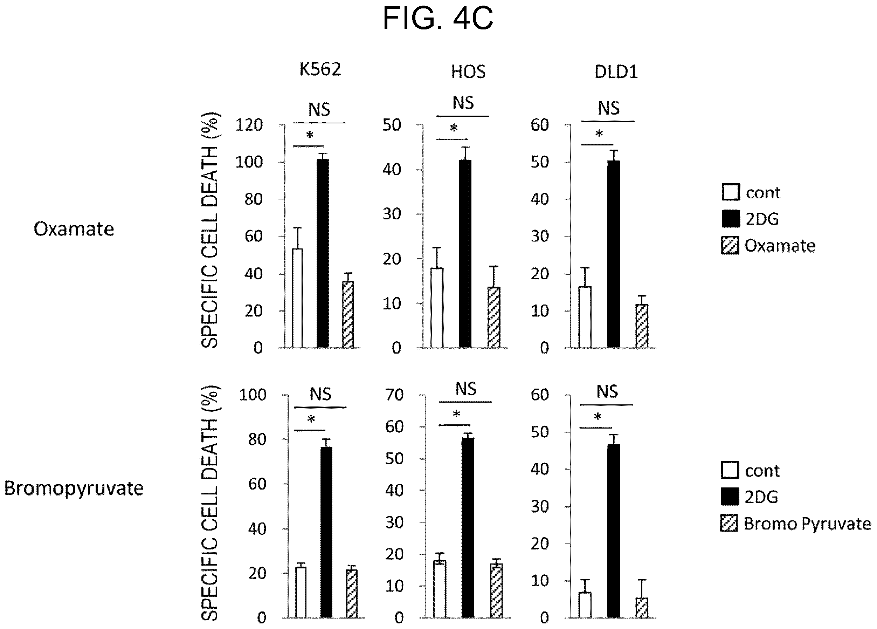

FIG. 4C shows the cytotoxic activity of the αβ T processed cells cultured with a glycolytic inhibitor (±) against cancer cells. NS: no significant difference; and * P < 0.01.

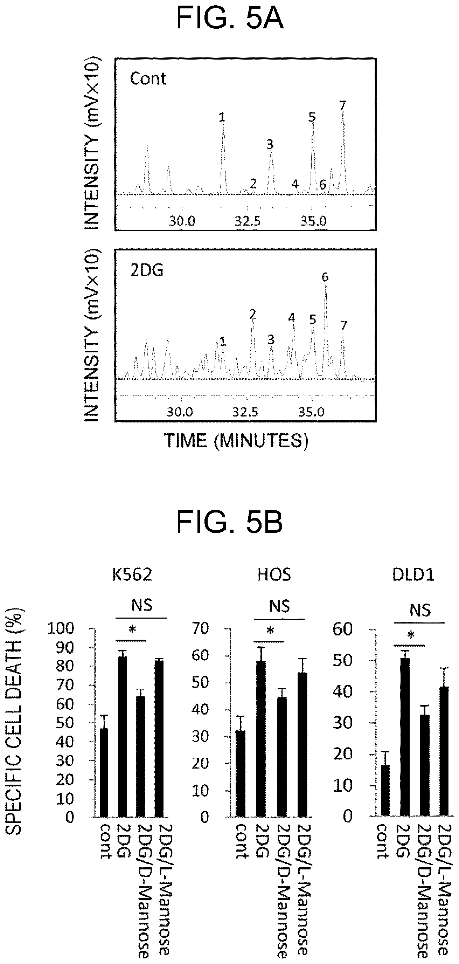

FIG. 5A shows the LC-MS mass spectrometry analysis of sugar chains on a cell surface.
FIG. 5B shows the suppression of cytotoxic activity by the addition of D-mannose to the αβ T cell culture in the presence of 2DG. The αβ T processed cells were cultured in the absence of 2DG (cont), in the presence of 2DG (2DG), in the presence of 2DG and D-mannose (2DG + D-Man), or in the presence of 2DG and L-mannose (2DG + L-Man). NS: no significant difference; and * P < 0.01.

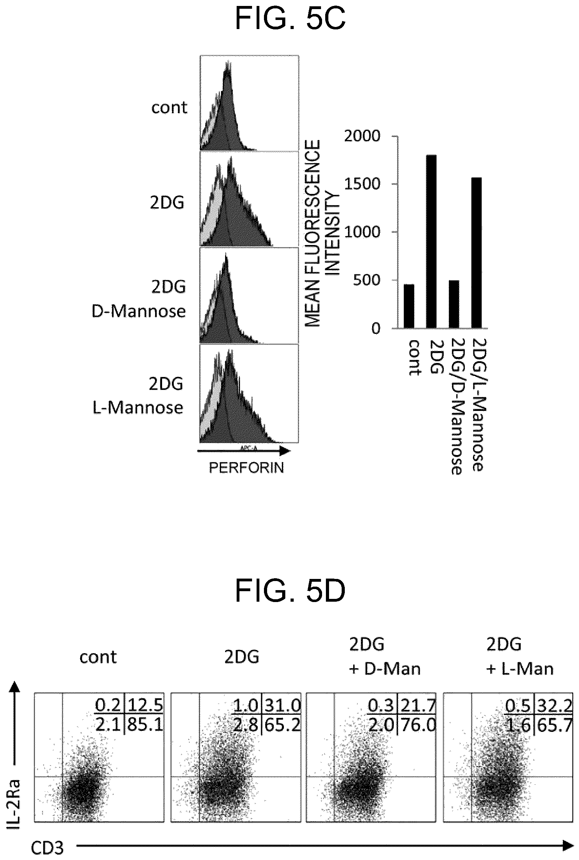

FIG. 5C shows the suppression of intracellular perforin production by the addition of D-mannose to the αβ T cell culture in the presence of 2DG. The αβ T processed cells were cultured in the absence of 2DG (cont), in the presence of 2DG (2DG), in the presence of 2DG and D-mannose (2DG + D-Man), or in the presence of 2DG and L-mannose (2DG + L-Man).
FIG. 5D shows FACS plots of IL-2 receptor expression in the αβ T processed cells cultured with 2DG. Each of the numbers in the drawing indicates the percentage of cells in each quadrant. The αβ T processed cells were cultured in the absence of 2DG (cont), in the presence of 2DG (2DG), in the presence of 2DG and D-mannose (2DG + D-Man), or in the presence of 2DG and L-mannose (2DG + L-Man).

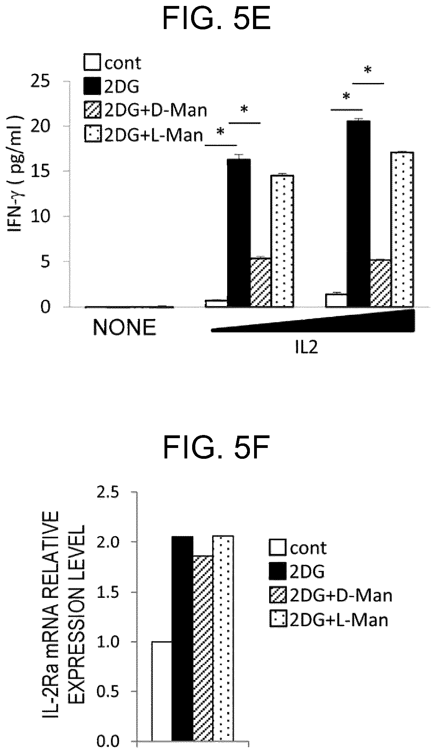

FIG. 5E shows the increase of IFNγ production by IL-2 restimulation on the αβ T processed cells cultured in the presence of 2DG (2DG). The amount was measured by an ELISA assay. The αβ T processed cells were cultured in the absence of 2DG (cont), in the presence of 2DG (2DG), in the presence of 2DG and D-mannose (2DG + D-Man), or in the presence of 2DG and L-mannose (2DG + L-Man). * P < 0.01
FIG. 5F shows the comparison of mRNA expression levels of IL-2 receptors in αβ T processed cells. The expression level was determined by using a real-time polymerase chain reaction (PCR) method. The αβ T processed cells were cultured in the absence of 2DG (cont), in the presence of 2DG (2DG), in the presence of 2DG and D-mannose (2DG + D-Man), or in the presence of 2DG and L-mannose (2DG + L-Man).

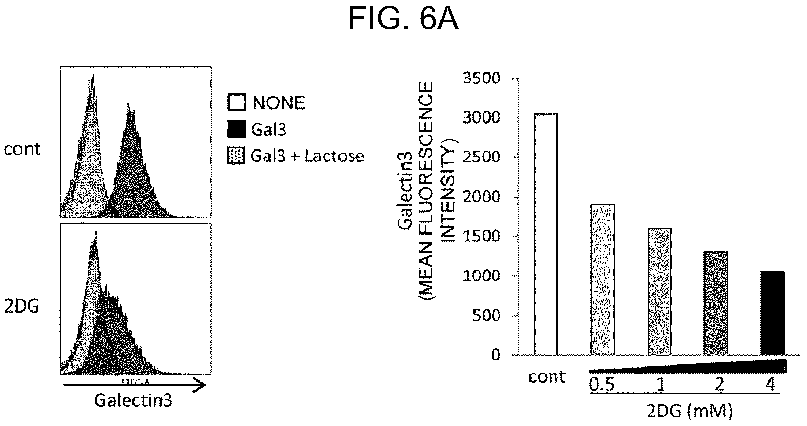

FIG. 6A shows the decrease of galectin-3 binding ability of the αβ T processed cells cultured in the presence of 2DG (2DG). The αβ T processed cells were cultured in the absence of 2DG (cont) or in the presence of 2DG (2DG).

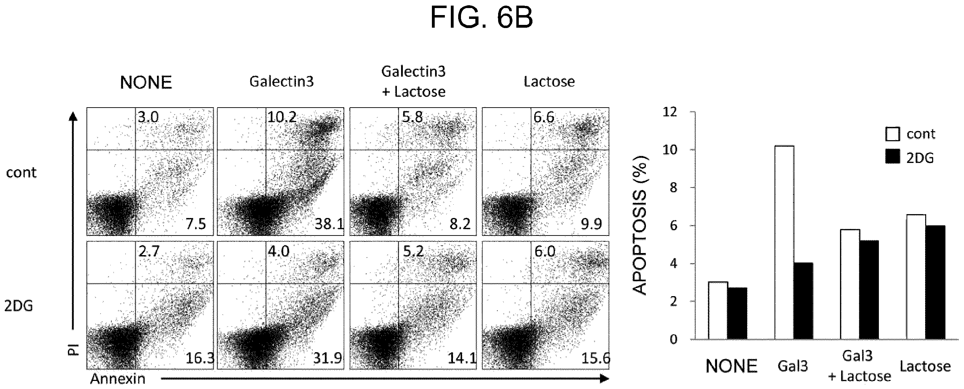

FIG. 6B shows the suppression of apoptosis (PI⁺/Annexin⁺) induced by galectin-3 against the αβ T processed cells cultured in the presence of 2DG (2DG). The αβ T processed cells were cultured in the absence of 2DG (cont) or in the presence of 2DG (2DG).

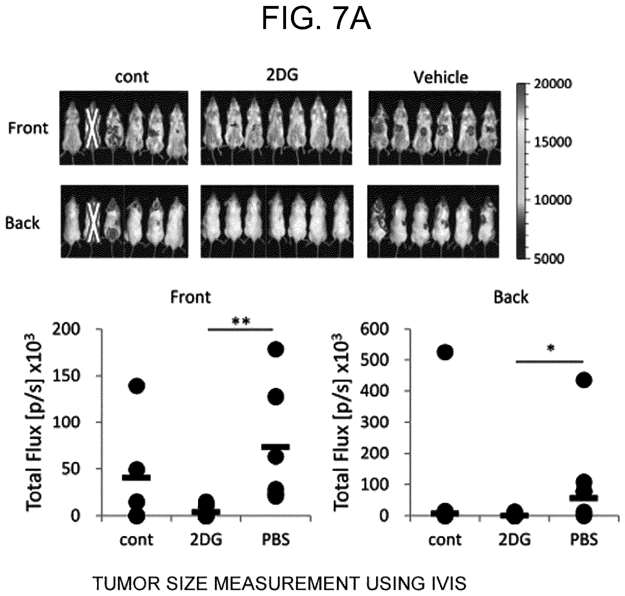

FIG. 7A shows the in-vivo anti-tumor effect by the αβ T processed cells treated with 2DG. The images are each tumor size imaging of the mice that have been intravenously injected with the αβ T processed cells cultured in the absence of 2DG (cont) or in the presence of 2DG (2DG), and intravenously injected with phosphate-buffered saline (PBS, vehicle).

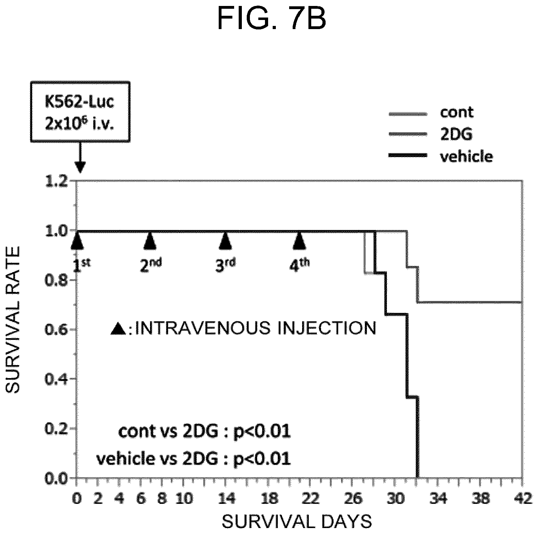

FIG. 7B shows the in-vivo anti-tumor effect by the αβ T cells treated with 2DG. The drawing shows the comparison of survival rates of the mice that have been intravenously injected with the αβ T processed cells cultured in the absence of 2DG (cont) or in the presence of 2DG (2DG), and intravenously injected with PBS (vehicle).

### DESCRIPTION OF EMBODIMENTS

Therefore, the present invention includes the following from [1] to [15]:
[1] A method for preparation of an αβ T processed cell, comprising
   in-vitro culturing an isolated peripheral blood mononuclear cell in the presence of
   (1) IL-2,
   (2) an anti-CD3 antibody, and
   (3) 2-deoxy-d-glucose (2DG) or a derivative thereof;
[2] The method according to [1], in which αβ T cells are contained at a proportion of around 74% to 90% and γδ T cells and NK cells are contained at a proportion of around 10% to 26%, in the αβ T processed cells;
[3] The method according to [1], in which CD8 positive memory T cells are contained at a proportion of 74, 75, 76, 77, or 78% or more in the αβ T processed cells;
[4] The method according to [1], in which IL-2R positive αβ T cells are contained at a proportion of 20, 21, 22, 23, 24, 25, 26, 27, 28, 29, or 30% or more, and/or perforin positive αβ T cells are contained at a proportion of 10, 11, 12, 13, 14, 15, 16, 17, 18, 19, or 20% or more and/or granzyme positive αβ T cells are contained at a proportion of 15, 16, 17, 18, 19, 20, 21, 22, 23, 24, or 25% or more, in the αβ T processed cells;
[5] The method according to any one of [1] to [4], in which a derivative or analog of 2DG is selected from the group consisting of 2-deoxymannose-6-phosphate, 2-deoxymannose-1-phosphate and GDP-2-deoxymannose, 2-fluoro-2-deoxyglucose, 2-fluorodeoxyglucose, 2-fluoro-deoxyglucose-6-phosphate, 2-fluoro-deoxyglucose-1-phosphate, 2-fluoro-deoxymannose, 2-fluoro-deoxymannose-6-phosphate, 2-fluoro-deoxymannose-1-phosphate, or a sugar having a six-membered ring such as galactose or mannose and a derivative thereof, and a physiologically acceptable salt or solvate thereof;
[6] A pharmaceutical composition comprising αβ T processed cells stimulated with 2-deoxy-d-glucose (2DG) or a derivative thereof, for the treatment or prevention of cancer;
[7] The pharmaceutical composition according to [6], in which the cancer expresses a NKG2D ligand;
[8] The pharmaceutical composition according to [7], in which the NKG2D ligand is selected from the group consisting of MICA, MICB, RAET1ε, RAET1α, RAET1β, RAET1γ, RAET1δ, Mult1, H60a, H60b, H60c, and ULBPs 1 to 6 MICA;
[9] The pharmaceutical composition according to [7], in which the cancer expressing the NKG2D ligand is selected from the group consisting of osteosarcoma, breast cancer, cervical cancer, ovarian cancer, endometrial cancer, melanoma, bladder cancer, lung cancer, pancreas cancer, colon cancer, prostate cancer, leukemia, acute lymphocytic leukemia, chronic lymphocytic leukemia, B-cell lymphoma, Burkitt's lymphoma, multiple myeloma, Hodgkin's lymphoma, non-Hodgkin's lymphoma, myeloid leukemia, acute myeloid leukemia (AML), chronic myeloid leukemia, thyroid cancer, follicular thyroid cancer, myelodysplastic syndrome (MDS), fibrosarcoma, rhabdomyosarcoma, melanoma, uveal melanoma, teratocarcinoma, neuroblastoma, glioma, glioblastoma, keratoacanthoma, kidney cancer, anaplastic large-cell lymphoma, esophageal squamous cell carcinoma, hepatocellular carcinoma, follicular dendritic cell carcinoma, intestinal cancer, muscle invasive cancer, testicular cancer, epidermal cancer, spleen cancer, head and neck cancer, gastric cancer, liver cancer, bone cancer, brain cancer, retinal cancer, biliary tract cancer, small intestinal cancer, salivary gland cancer, uterine cancer, testicular cancer, connective tissue cancer, benign prostatic hyperplasia, myelodysplasia, waldenstrom's macroglobulinemia, nasopharyngeal cancer, neuroendocrine carcinoma, mesothelioma, angiosarcoma, Kaposi's sarcoma, carcinoid, esophageal gastric cancer, fallopian tube cancer, peritoneal cancer, serous papillary Muller tube cancer, malignant ascites, gastrointestinal stromal tumor (GIST), and Li-Fraumeni syndrome;
[10] A pharmaceutical composition comprising αβ T processed cells stimulated with 2-deoxy-d-glucose (2DG) or a derivative thereof, for the treatment or prevention of infection with a bacterium or a virus;
[11] The pharmaceutical composition according to [10], in which the bacterium is selected from a Gram-negative bacterium or a Gram-positive bacterium;
[12] The pharmaceutical composition according to [10], in which the virus is selected from the group consisting of cytomegalovirus, influenza virus, Epstein-Barr virus (EBV), adenovirus, hepatitis B virus, hepatitis C virus, human papillomavirus, human T-lymphotropic virus (HTLV), and human immunodeficiency virus (HIV);
[13] Use of the αβ T processed cells prepared by the method according to any one of [1] to [5] in production of a product for regenerative medicine or the like;
[14] The use according to [13], in which the product for regenerative medicine or the like is a product for regenerative medicine or the like for the treatment or prevention of cancer; and
[15] The use according to [13], in which the product for regenerative medicine or the like is a product for regenerative medicine or the like for the treatment or prevention of infection with a bacterium or a virus.

### <1. Production Method for αβ T processed cells>

First, peripheral blood mononuclear cells (PBMCs) as a cell source for αβ T cells are prepared. Herein, the expression "peripheral blood mononuclear cells (PBMCs)" means a cell population containing lymphocytes (such as natural killer cells, natural killer T cells, αβ T cells, and γδ T cells), monocytes, and the like, which are isolated from the peripheral blood. The method for preparing PBMCs is not particularly limited. For example, the PBMCs can be obtained by subjecting the peripheral blood obtained by blood collection to density gradient centrifugation. The volume of collected blood at one time may be appropriately set depending on the patient to be subjected to the αβ T cell therapy, but is, for example, around 24 to 72 mL.

In addition, in a case where a large amount of cells are required, it is possible to collect mononuclear cell components by using a blood component collection device.

The collected PBMCs are suspended in a culture solution (medium), and into the obtained cell suspension, IL-2, anti-CD3 antibodies, and 2DG are added to culture αβ T cells. By culturing the PBMCs in the presence of IL-2 and anti-CD3 antibodies, the αβ T cells are selectively expanded and activated, and a cell population containing the activated αβ T cells in high purity can be prepared. In the conventional production of αβ T processed cells without the addition of 2DG, around 94% is T cells, around 6% is NK cells, and a cell population containing T cells around 86% of which is αβ T cells (around 69% of CD8 positive T cells + around 31% of CD4 positive T cells); and around 14% of which is γδ T cells was prepared.

The anti-CD3 antibodies may be added to a medium or immobilized on a culture container, but it is known that more suitable culture can be achieved by inoculating lymphocytes in a culture container such as a flask on which the anti-CD3 antibodies are immobilized (for example, JP-B-3056230). It is preferable that the IL-2 is added to a medium so that the concentration of IL-2 is 100 to 2000 IU/mL.

The culture is conducted at 34 to 38°C and preferably 37°C under the condition of CO₂ of 2 to 10% and preferably 5%, and the culture period is 1 to 20 days, and particularly preferably around 1 to 2 weeks.

The medium to be used is not particularly limited, and a commercially available medium used for cell culture, such as AIM-V medium (Invitrogen), RPMI-1640 medium (Invitrogen), Dulbecco's modified Eagle's medium (Invitrogen), Iscove's medium (Invitrogen), KBM medium (Kohjin Bio Co., Ltd.), or ALyS medium (Cell Science & Technology Institute, Inc.) can be used. As the medium, a medium for human peripheral blood T cells (for example, ALyS505N: Cell Science & Technology Institute, Inc.), in which IL-2 has been contained beforehand, may also be used. Furthermore, into the medium, 5 to 20% of bovine serum, fetal bovine serum (FBS), human serum, human plasma, or the like can be added as needed.

In the present invention, the expression "2-deoxy-d-glucose" refers to a compound represented by the following formula, or a pharmaceutically acceptable salt or a solution thereof.

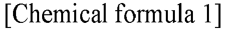

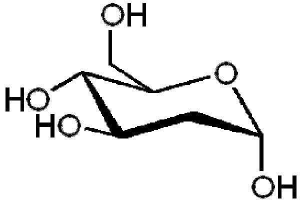

Examples of the compound capable of being used in the same way as 2DG include a publicly known derivative of 2DG, and an analog thereof. Examples of the compound include, but are not limited to, 2-deoxymannose-6-phosphate, 2-deoxymannose-1-phosphate and GDP-2-deoxymannose, 2-fluoro-2-deoxyglucose, 2-fluorodeoxyglucose, 2-fluoro-deoxyglucose-6-phosphate, 2-fluoro-deoxyglucose-1-phosphate, 2-fluoro-deoxymannose, 2-fluoro-deoxymannose-6-phosphate, 2-fluoro-deoxymannose-1-phosphate, or a sugar having a six-membered ring such as galactose or mannose, and a derivative thereof. The above compound may be a free body, or a physiologically acceptable salt or a solvate thereof. Furthermore, the compound may also be a hydrate, or a non-hydrate. Examples of the salt include, but are not limited to, an inorganic salt such as a hydrochloride, and an organic acid salt such as an acetate, a citrate, or a formate.

The concentration of the drug to be added at the time of culturing PBMCs is preferably 5 mM or less, and the drug is used at a concentration of 2 mM in Examples herein.

By the above procedure, a cell population containing a large amount of αβ T processed cells can be obtained. The obtained cell population can be used as the cells to be administered to a patient in αβ T cell therapy as described later. Furthermore, the cell population can also be used as a product for regenerative medicine or the like.

The expression "product for regenerative medicine or the like" refers to:
1) a product, which is obtained by applying culture and other processing to human or animal cells, among the products that are intended to be used for
   (i) the reconstruction, repair, or formation of the structure or function of a human or animal body, or
   (ii) the treatment or prevention of human or animal diseases; or
2) a product, which is introduced into a human or animal cell and contains a gene to be expressed in the body thereof, among the products that are intended to be used for the treatment of human or animal diseases. Examples of the product for regenerative medicine or the like include, but are not particularly limited to,
   1) a human cell processed product,
      (a) a human somatic cell processed product,
      (b) a human somatic stem cell processed product,
      (c) a human embryonic stem cell processed product, or
      (d) a human induced pluripotent stem cell processed product;
   2) an animal cell processed product,
      (a) an animal somatic cell processed product,
      (b) an animal somatic stem cell processed product,
      (c) an animal embryonic stem cell processed product, or
      (d) an animal induced pluripotent stem cell processed product; and
   3) a product for gene therapy,
      (a) a plasmid vector product,
      (b) a virus vector product, or
      (c) a gene expression therapy product (excluding (a) and (b)). The cell population containing a large amount of the αβ T processed cells in the present invention falls into the category of the human somatic cell processed product.

NKG2D (also known as CD314, or Killer Cell Lectin Like Receptor K1 (KLRK1)) is a cell surface receptor (type II transmembrane protein) of the NKG2D family, and is expressed on a NK cell, a CD8⁺ αβ T cell, and a γδ T cell. When a ligand is bound to the NKG2D, the NKG2D induces the cytotoxic activity of NK cells and the conjugate stimulation on a specific T cell population via the associated adaptor protein DAP10, DAP12 or the like. The ligand of the NKG2D is a stress-inducible MHC class I-related molecule, such as MIC A, MIC B, RAET1E, RAET1G, ULBP1, ULBP2, ULBP3, ULBP4 or the like, and such a ligand is mainly expressed in an epithelial cell, or numerous cancer cells. The expression of the NKG2D ligand is induced on a cell surface by cellular stress other than cancer cells. Accordingly, the expression is induced by not only cancer cells, but also other cellular stress, for example, bacterial infection, viral infection, or the like, and the cells infected with bacteria or viruses may be targets of the αβ T processed cells according to the present invention (NON-PATENT LITERATURE 5).

Examples of the cancer cell expressing the NKG2D ligand include, but are not limited to, cells of osteosarcoma, breast cancer, cervical cancer, ovarian cancer, endometrial cancer, bladder cancer, lung cancer, pancreas cancer, colon cancer, prostate cancer, leukemia, acute lymphocytic leukemia, chronic lymphocytic leukemia, B-cell lymphoma, Burkitt's lymphoma, multiple myeloma, Hodgkin's lymphoma, non-Hodgkin's lymphoma, myeloid leukemia, acute myeloid leukemia (AML), chronic myeloid leukemia, thyroid cancer, follicular thyroid cancer, myelodysplastic syndrome (MDS), fibrosarcoma, rhabdomyosarcoma, melanoma, uveal melanoma, teratocarcinoma, neuroblastoma, glioma, glioblastoma, keratoacanthoma, kidney cancer, anaplastic large-cell lymphoma, esophageal squamous cell carcinoma, hepatocellular carcinoma, follicular dendritic cell carcinoma, intestinal cancer, muscle invasive cancer, testicular cancer, epidermal cancer, spleen cancer, bladder cancer, head and neck cancer, gastric cancer, liver cancer, bone cancer, brain cancer, retinal cancer, biliary tract cancer, small intestinal cancer, salivary gland cancer, uterine cancer, testicular cancer, connective tissue cancer, benign prostatic hyperplasia, myelodysplasia, waldenstrom's macroglobulinemia, nasopharyngeal cancer, neuroendocrine carcinoma, myelodysplastic syndrome, mesothelioma, angiosarcoma, Kaposi's sarcoma, carcinoid, esophageal gastric cancer, fallopian tube cancer, peritoneal cancer, serous papillary Muller tube cancer, malignant ascites, gastrointestinal stromal tumor (GIST), and Li-Fraumeni syndrome.

Examples of the bacterium inducing the NKG2D ligand by infection include, but are not limited to, a Gram-negative bacterium such as E. coli, and a Gram-positive bacterium such as Mycobacterium.

Examples of the virus inducing the NKG2D ligand by infection include, but are not limited to, cytomegalovirus, influenza virus, Epstein-Barr virus (EBV), adenovirus, hepatitis B virus (HBV), hepatitis C virus (HCV), human papillomavirus, human T-lymphotropic virus (HTLV), and HIV.

### <2. Medicament containing αβ T processed cells>

The medicament of the present invention is a medicament containing the αβ T processed cells that are produced by the above-described production method of the present invention, for the treatment or prevention of cancer.

The medicament of the present invention is, for example, an injection (cell suspension) that is prepared by suspending the αβ T processed cells produced by the production method of the present invention in a liquid (for example, physiological saline solution) capable of being used as a medicament. This injection may be injected intravenously, intradermally, subcutaneously, or the like, may be directly injected into a lesion site, or may be administered systemically as an infusion. The medicament of the present invention may contain a physiologically acceptable carrier or excipient.

The medicament of the present invention contains the αβ T cells produced by the production method of the present invention as an essential component, and may contain other components as an optional component. For example, the medicament of the present invention may contain a cytokine such as IL-2, and may also contain an anticancer agent and the like. These drugs may be administered simultaneously, or may be administered continuously at regular intervals.

Examples of the anticancer agent include, but are not limited to, gemcitabine, 5-FU, cisplatin, docetaxel, paclitaxel, zoledronic acid, and an immune checkpoint inhibitor.

The number of the αβ T processed cells contained in the medicament of the present invention can be appropriately set depending on the administration method, the kind of disease, the symptom of the patient, and the like. In general, the number of the αβ T processed cells may be set so as to be 10⁸ to 10¹² cells/person (preferably 10⁹ cells/person).

The production method of the medicament of the present invention is not particularly limited. For example, the medicament of the present invention can be produced as an infusion capable of being administered intravenously by: 1) collecting the αβ T processed cells obtained by the production method for αβ T processed cells according to the present invention by centrifugation or the like; 2) washing the collected αβ T processed cells with a washing solution (for example, physiological saline solution, or PBS); 3) collecting the washed αβ T processed cells by centrifugation or the like; and 4) suspending the recovered αβ T processed cells in a liquid (for example, physiological saline solution) capable of being used as a medicament.

Hereinafter, the present invention will be described in detail by way of Examples; however, the present invention is not limited to the following Examples.

### Examples

### Example 1: Preparation of αβ T processed cells in the presence of 2DG

Blood samples were collected after approval by ethics review boards of all cooperating institutions. Peripheral blood mononuclear cells (PBMCs) were isolated from the blood of healthy volunteer donors. The PBMCs were cultured for 2 weeks in an ALyS medium (Cell Science & Technology Institute, Inc.) containing 175 IU/ml of IL-2 supplemented with autologous plasma or serum in a culture container in which anti-CD3 monoclonal antibodies (Janssen Pharmaceutical K.K.) were immobilized. In the culture, 2 mM of 2DG (Sigma-Aldrich Co. LLC) was added.

### Example 2: Distribution analysis of CD8⁺ T-cell subset using flow cytometry

The αβ T processed cells prepared in Example 1 were analyzed by using a flow cytometer FACS CantoII (BD Biosciences). After washing the cells with PBS, the washed cells were centrifuged, and the obtained cells were resuspended in PBS containing 2% FBS and 5 mM of ethylenediaminetetraacetic acid (EDTA). The obtained resuspension was passed through a cell strainer to remove aggregates. Thus obtained material was incubated for 1 hour on ice, together with phycoerythrin (PE)-labeled anti-CD8 antibodies (Beckman Coulter, Inc.), fluorescein isothiocyanate (FITC)-labeled anti-CCR7 antibodies, and PE/Cy5-labeled anti-CD45RO antibodies (BioLegend, Inc.), and after the washing of the incubated material, the measurement was performed. The data were analyzed by using Kaluza (Beckman Coulter, Inc.) software (FIG. 1).

As a result, the αβ T processed cells treated with 2DG showed the phenotype of memory T cells (CCR7±CD45RO+).

The immature T cells (naive T cells) are lymphocytes that have not yet been stimulated with antigens and have expressed CD45RA antigens on the cell surfaces thereof, and are activated by encountering antigen presenting cells such as dendritic cells, so that they become effector T cells.

The effector T cells express CD45RO antigens in place of the CD45RA antigens on the cell surfaces thereof. Furthermore, some of the activated T cells (effector T cells) become memory T cells after antigens such as pathogens are eliminated.

The memory T cells are lymphocytes that have already been stimulated with antigens and expressed the CD45RO antigens, regardless of whether the stimulation is specific stimulation or non-specific stimulation, and are maintained in the body for a long period of time while maintaining the memory of specific or non-specific antigens. The memory T cells can be divided into effector memory (EM) T cells (CCR7 negative CD45RO positive), and central memory (CM) T cells (CCR7 positive CD45RO positive).

Accordingly, a pharmaceutical preparation containing a memory T cell group as a main component is maintained in the body for a long period of time, and thus has a high possibility of obtaining a high therapeutic effect in immune cell therapy.

### Example 3: Cytotoxic activity of αβ T processed cells treated with 2DG

### 3.1. Secretion of cytokines (IL-2, IFN-γ, and TNFα), and perforin and granzyme B

The cytokine production ability of the αβ T processed cells prepared in Example 1 was determined by using Bio-plex "human cytokine G1 27 plex panel" (Bio-Rad Laboratories, Inc.) (FIG. 2A). Specifically, the αβ T processed cells prepared in Example 1 were replaced in a medium containing RPMI 1640 and 10% FCS in the absence of IL-2, and cultured. The culture supernatant after 24 hours from the start of the culture was recovered, and subjected to measurement.

Furthermore, perforin and granzyme B in the culture supernatant after the degranulation due to the PMA (5 ng/ml) and ionomycin (0.5 µg/ml) stimulation were determined by using Human Perforin ELISA Kit and Human Granzyme B ELISA Kit (Abcam plc.), respectively (FIG. 2B).

As a result, secretions of cytokines, perforin, and granzyme B were all increased by 2DG treatment in 3 cases out of 4 cases of healthy donor samples.

IL-2 activates T cells, IFN-γ and TNFα are produced also from the T cells, and an anti-tumor effect is exerted. Perforin is a glycoprotein that is present in cytoplasmic granules of a killer T cell or a NK cell, and causes cytotoxicity by entering the membrane lipid of a target cell and making a hole in there. Granzyme B, which is one type of serine proteases, invades the inside of a target cell via perforin activates the caspase cascade to induce apoptosis or necrosis in the target cell.

Accordingly, it was suggested that the cytotoxic activity of the αβ T processed cells treated with 2DG was higher as compared with that of the conventional method.

### 3.2. In vitro cytotoxic activity against cultured cells

The cytotoxic activity test by fluorescent staining was performed by using Terascan VPC2 (Minerva Tech K.K.). Cancer cell line K562 (leukemia cell line), Daudi (Burkitt's lymphoma cell line), and HOS (osteosarcoma cell line), and DLD1 (colon cancer cell line) were labeled for 30 minutes with 2.55 to 5 µg/mL of fluorescent dye Cellstein R-Calcein-AM solution (DOJINDO LABORATORIES). The labeled cancer cells and the αβ T processed cells were mixed at a ratio of 1 : 25, and the mixed cells were incubated for 2 to 4 hours in RPMI 1640 and 10% FBS (in the absence of IL-2). The cytotoxic activity was calculated by using changes in the fluorescence intensity of the cancer cells co-cultured with the αβ T processed cells as an index, assuming the fluorescence decay rate when treated with 1% NP40 (surfactant) as 100% (FIG. 2C).

As a result, it was indicated that the αβ T processed cells treated with 2DG had a significantly increase in the cytotoxic activity against the cells other than Daudi cells, as compared with that of the conventional method.

### Example 4: Relationship between cytotoxicity and degranulation

In the αβ T processed cells treated with 2DG as described above, secretions of effector factors such as cytokines, perforin, and granzyme B were promoted, and the cytotoxic activity was enhanced. Since exposure of CD107a localized in an intracellular vesicle onto the cell surface serves as an index of degranulation for the release of an effector factor from a cytotoxic T cell or a NK cell, the exposure of CD107a onto the cell surface by co-culture with cancer cells having different cytotoxicity was detected.

In the presence of APC-labeled anti-CD107a antibodies (BioLegend, Inc.), the αβ T processed cells and cancer cells were mixed at a ratio of 25: 1, and the mixed cells were cultured for 6 hours. After the washing of the cultured cells, the washed cells were stained with PE-labeled anti-CD8 antibodies (Beckman Coulter, Inc.). The cells thus stained were subjected to measurement by using a flow cytometer FACS CantoII (BD Biosciences), and the obtained data were analyzed by using Kaluza (Beckman Coulter, Inc.) software (FIG. 2D).

As a result, in the αβ T processed cells co-cultured with K562 cells the cytotoxic activity of which had been increased due to the 2DG treatment, the exposure of CD 107a onto the cell surface (that is, degranulation of CD8⁺ T cells) was promoted, and in the αβ T processed cells co-cultured with Daudi cells the cytotoxic activity of which had not been increased, the exposure of CD 107a onto the cell surface was not promoted.

### Example 5: Dependency of NKG2D and NKG2D ligands

From the relationship between the cytotoxic activity and the degranulation, it was considered that the cytotoxic activity that had directly recognized cancer cells might be enhanced by the 2DG treatment. Therefore, the cytotoxicity mediated by NKG2D expressed on a T cell and a NKG2D ligand expressed on the cancer cell side was confirmed.

### 5.1. Expression level of NKG2D

By using a flow cytometer FACS CantoII (BD Biosciences), the expression of NKG2D on a surface of a CD8⁺ T cell was analyzed. The cells were washed with PBS, and then the washed cells were centrifuged, and the obtained cells were resuspended in PBS containing 2% FBS and 5 mM of ethylenediaminetetraacetic acid (EDTA). The obtained resuspension was passed through a cell strainer to remove aggregates. The resultant material was incubated for 1 hour on ice, together with PE-labeled anti-NKG2D antibodies, FITC-labeled anti-CD3 antibodies, and PC5-labeled anti-CD8 antibodies (Beckman Coulter, Inc.), and after the washing of the incubated material, the measurement was performed. The data were analyzed by using Kaluza (Beckman Coulter, Inc.) software (FIG. 3A).

As a result, no difference was observed in the NKG2D expression of the αβ T processed cells treated with 2DG (±).

### 5.2. Expression of NKG2D ligands on cancer cells

By using a flow cytometer FACS CantoII (BD Biosciences), the expression of the NKG2D ligands on a surface of each of various kinds of cancer cells was analyzed. After each of the cultured cancer cells was washed with PBS, the washed cells were centrifuged, and the obtained cells were resuspended in PBS containing 2% FBS and 5 mM of ethylenediaminetetraacetic acid (EDTA). The obtained resuspension was passed through a cell strainer to remove aggregates. The obtained cancer cells were incubated for 1 hour on ice, together with anti-MIC A/B antibodies (BioLegend, Inc.), anti-ULBP1 antibodies, anti-ULBP2/5/6 antibodies, and anti-ULBP3 antibodies (R&D Systems, Inc.). After the washing of the incubated cancer cells, the cancer cells were reacted with FITC-labeled anti-mouse IgG antibodies (Jackson ImmunoResearch Laboratories, Inc.) on ice for 30 minutes, and the obtained cancer cells were subjected to washing, and then the measurement was performed. The data were analyzed by using Kaluza (Beckman Coulter, Inc.) software (FIG. 3B).

As a result, high expression of NKG2D ligands was observed in the cells other than Daudi cells, and it was suggested that the binding of NKG2D and a NKG2D ligand was related to the cytotoxic activity.

### 5.3. Suppression of cytotoxic activity by anti-NKG2D antibody

In order to examine the involvement of the NKG2D on an αβ T processed cell in the cytotoxic ability against cancer, the αβ T processed cells were reacted for 60 minutes with anti-human NKG2D blocking antibodies (R&D Systems, Inc.) or mouse IgG1 (BioLegend, Inc.), which is isotype antibody, as a control, before co-culturing of the αβ T processed cells with cancer cells. The cytotoxic activity was determined in accordance with the description of 3.2 (FIG. 3C).

As a result, by blocking the NKG2D, the cytotoxic activity of the αβ T processed cells treated with 2DG was remarkably decreased except for that of Daudi cells.

### Example 6: Relationship between cytotoxic activity and glycolytic control

In order to verify whether or not the effect of 2DG was due to the glycolytic inhibition of the αβ T processed cells, it was verified whether or not an effect similar to that of 2DG was exerted with the addition of a glycolytic inhibitor other than 2DG, such as oxamate and bromo pyruvate (Sigma-Aldrich Co. LLC).

### 6.1. FACS analysis with addition of glycolytic inhibitor

When the αβ T processed cells of Example 1 were prepared, oxamate (2 mM) or bromo pyruvate (10 µM), which is a glycolytic inhibitor, was added in place of 2DG, and the αβ T processed cells were prepared. The prepared αβ T processed cells were subjected to distribution analysis of CD8⁺ T-cell subset by the method described in Example 2.

As a result, in the αβ T processed cells treated with oxamate (2 mM) or bromo pyruvate (10 µM), which is a glycolytic inhibitor, the phenotype that was observed when treated with 2-DG was not indicated (FIG. 4A).

### 6.2. Evaluation of glycolytic capacity

Since the 2DG is a glycolytic inhibitor, when the αβ T processed cells were prepared, oxamate or bromo pyruvate, which is a glycolytic inhibitor other than 2DG, was added in place of 2DG, and the αβ T processed cells were prepared. Then the glycolytic capacity of the cells was evaluated.

By using XF Glycolysis Stress Test Kit (Agilent Technologies, Inc.), the glycolytic capacity, which is the main energy metabolic pathway of cells, was evaluated. In the measurement, by monitoring the changes in the extracellular hydrogen ion concentration (mpH/min), Extracellular Flux Analyzer XF (Agilent Technologies, Inc.) was used to calculate the glycolysis system ≈ extracellular acidification rate (ECAR). For the analysis, Wave (Agilent Technologies, Inc.) software was used.
1) Glycolysis: showed glycolytic capacity in steady state.
2) Glycolytic Capacity: showed maximum glycolytic capacity possessed by cells.

As a result, it was confirmed that the glycolysis systems of the αβ T processed cells treated with 2DG and the αβ T processed cells treated with each inhibitor were similarly inhibited (FIG. 4B).

### 6.3. In vitro cytotoxic activity against cultured cells

By using the method described in 3.2. of Example 3, the cytotoxic activity against each of cancer cells, by the αβ T processed cells treated with oxamate (2 mM) or bromo pyruvate (10 µM), which is a glycolytic inhibitor, was examined.

As a result, in the αβ T processed cells treated with oxamate or bromo pyruvate, no enhancement of the cytotoxic activity was observed as compared with the cells not treated with 2DG (FIG. 4C).

Accordingly, it was suggested that the mechanism other than the glycolytic inhibition was involved in the enhancement of the cytotoxic activity of the αβ T processed cells treated with 2DG.

### Example 7: Relationship between cytotoxic activity and glycosylation

It is known that 2DG inhibits the addition reaction of mannose consisting of N-linked glycans by 2DG's having a structure similar to that of the mannose. Therefore, in order to verify whether or not the effect of 2DG was due to the influence on the glycosylation of the αβ T processed cells, the changes in the sugar chains of the αβ T processed cells treated with 2DG were verified. Furthermore, it was verified whether or not the effect of 2DG were suppressed with the addition of mannose.

### 7.1. LC-MS mass spectrometry analysis of sugar chains on cell surface

From the αβ T processed cells, sugar chains were purified and labeled by using BlotGlyco (Sumitomo Bakelite Co., Ltd.), and then the purified and labeled sugar chains were subjected to LC-MS mass spectrometry analysis (FIG. 5A). The amount of sugar chains (fmol) per cell of the peak number and the estimated structure of sugar chains are shown in Table 1.

**[Table 1]**

| Peak | Estimated glycan composition | Control | 2 D G | Proportion |
|---|---|---|---|---|
| | | | | (2DG/Control) |
| 1 | (HexNAc)2(Sulph)1 + (Man)3(GlcNAc)2 | 420. 4 | 233.7 | 0.56 |
| 2 | (Hex)2(HexNAc)3(Deoxyhexose)1+ (Man)3(GlcNAc)2 | 40.5 | 483.4 | 11.94 |
| 3 | (Hex)1(HexNAc)2(Sulph)1 + (Man)3(GleNAc)2 | 351.5 | 281.5 | 0.8 |
| 4 | (Hex)1(HexNAc)1(Deoxyhexose)1(NeuGc)1 + (Man)3(GlcNAc)2 | 28.2 | 420 | 14.89 |
| 5 | (Hex)2(HexNAc)2(Sulph)1 + (Man)3(GlcNAc)2 | 449.4 | 605 | 1.35 |
| 6 | (Hex)2(HexNAc)2(Deoxyhexose)1(Sulph)1 + (Man)3(GlcNAc)2 | 39.5 | 605 | 15.34 |
| 7 | (HexNAc)4(Sulph)2 + (Man)3(GlcNAc)2 | 543.7 | 323 | 0.59 |

As a result of sugar chain analysis, the effect of 2DG treatment indicated mainly abnormal increase of the mature branched N-glycan in which deoxyhexose had been introduced rather than suppression of synthesis of (Hex)3(Man)9(GlcNAc)2, which is a precursor of N-glycan. Accordingly, it was suggested that the N-glycosylated surface proteins of the αβ T processed cells treated with 2DG had altered affinities for various ligands.

### 7.2. Suppression of cytotoxic activity of αβ T processed cells treated with 2DG by D-mannose

It has been reported that 2DG competitively inhibits the addition of mannose during glycosylation. Therefore, it was investigated whether or not the cytotoxic activity ability was suppressed by adding D-mannose to the processing of αβ T cells treated with 2DG.

In the preparation of Example 1, αβ T processed cells were prepared with the addition of 2 mM of D-mannose (Sigma-Aldrich Co. LLC) or L-mannose (Tokyo Chemical Industry Co., Ltd.), and the prepared αβ T processed cells were subjected to the measurement of cytotoxic activity by the method described in item 3.2.

As a result, it was found that the addition of D-mannose reduced the effect of 2DG. On the other hand, even when L-mannose, which is an isomer of D-mannose, was added, there was no influence on the cytotoxic activity of 2DG (FIG. 5B).

### 7.3. Suppression of intracellular perforin production in αβ T processed cells treated with 2DG by D-mannose

Next, it was verified whether or not mannose inhibited the increase in the amount of intracellular perforin of the αβ T processed cells treated with 2DG. In the preparation of Example 1, αβ T processed cells were prepared with the addition of 2 mM of D-mannose (Sigma-Aldrich Co. LLC) or L-mannose (Tokyo Chemical Industry Co., Ltd.), the prepared cells were treated with IntraPrep Permeabilization Reagent (Beckman Coulter, Inc.), and the expression level of the intracellular perforin was determined by staining the cells with APC-labeled anti-perforin antibodies. The stained cells were subjected to measurement by using a flow cytometer FACS CantoII (BD Biosciences), and the data were analyzed by using Kaluza (Beckman Coulter, Inc.) software. The Mean Fluorescence Intensity (MFI) value is mean fluorescence intensity, and reflects the expression level.

As a result, it was found that the addition of D-mannose reduced the effect of 2DG. On the other hand, there was no influence by the addition of L-mannose (FIG. 5C). Furthermore, although the data are not shown, the results were also similar to those for intracellular granzyme B.

### 7.4. Induction of IL-2 receptor expression by 2DG

It is known that the intracellular perforin and granzyme B in effector cells are induced and expressed by the stimulation from IL-2 receptor on a T cell. Therefore, the expression of the IL-2 receptor was confirmed. The αβ T processed cells prepared in item 7.3. were washed with PBS, and then the washed cells were centrifuged, and the obtained cells were resuspended in PBS containing 2% FBS and 5 mM of EDTA. The obtained resuspension was passed through a cell strainer to remove aggregates. The obtained cells were incubated for 1 hour on ice, together with PE/Cy5-labeled anti-IL-2R antibodies (BioLegend, Inc.), and FITC-labeled anti-CD3 antibodies (Beckman Coulter, Inc.), and after the washing of the incubated cells, the measurement was performed by using a flow cytometer FACS CantoII (BD Biosciences). The data were analyzed by using Kaluza (Beckman Coulter, Inc.) software.

As a result, the expression of the IL-2 receptor on a cell surface was increased in the αβ T processed cells treated with 2DG, and it was suggested that such expression was suppressed by D-mannose (FIG. 5D).

### 7.5. Induction of interferon-γ secretion

From the viewpoint that the expression of the IL-2 receptor in αβ-processed cells cultured with 2DG was increased, it was confirmed whether or not the cell response mediated by IL-2 was also increased. The αβ T processed cells cultured in the absence of 2DG (cont), in the presence of 2DG (2DG), in the presence of 2DG and D-mannose (2DG + D-Man), or in the presence of 2DG and L-mannose (2DG + L-Man) were collected, the collected cells were precultured for 24 hours in RPMI 1640 medium and 10% FCS in the absence of IL-2, and then the precultured cells were again cultured in RPMI 1640 medium and 10% FCS with IL-2 (0, 50, or 250 IU/ml, NIPRO CORPORATION). The culture supernatant after the lapse of 24 hours from the start of the culture was collected, and yield of the IFN-γ was determined by ELISA Kit (Abcam plc.) (FIG. 5E).

### 7.6. Induction of IL-2 receptor gene expression by 2DG

It was confirmed whether the changes in the expression level of IL-2 receptor by 2DG were controlled at the protein expression level or at the gene expression level. RNA was extracted from the αβ T processed cells cultured in the absence of 2DG (cont), in the presence of 2DG (2DG), in the presence of 2DG and D-mannose (2DG + D-Man), or in the presence of 2DG and L-mannose (2DG + L-Man), and by using the extracted RNA, cDNA was synthesized with a reverse transcriptase (Superscript III, Thermo Fisher Scientific K.K.). The gene expression level of IL-2 receptor α was determined by a real-time PCR system ViiA7 (Applied Biosystems) using the cDNA under each condition. The expression level of IL-2 receptor α in each sample was corrected with endogenous control β-actin, and then the relative value was graphed by assuming the cont as 1 (FIG. 5F).

### Example 8: Suppression of cytotoxic activity by galectin-3

Galectin-3, which is a member of the galectin family that has one or more of carbohydrate recognition domains with an affinity for β-galactoside, is secreted from a cancer cell, and not only induces angiogenesis but also binds to a glycosylated receptor on a CD8 positive T cell to induce unresponsiveness or apoptosis of the cell. Since the effect of 2DG is allowed to alter the glycosylation on a cell surface of the αβ T processed cell, it was examined whether or not there was a change in the influence of galectin-3 on the αβ T processed cell treated with 2DG.

### 8.1. Galectin-3 binding ability

It was verified whether or not the αβ T processed cell treated with 2DG (±) binds to galectin-3. Recombinant galectin-3 (BBI Solutions) was added to the αβ T processed cells cultured with the addition of 0 to 4 mM of 2DG, the obtained cells were incubated at 4°C for 30 minutes, and then the incubated cells were reacted with anti-galectin-3 antibodies (LifeSpan BioSciences Inc.). The galectin-3 binding ability was detected with FITC-labeled anti-mouse IgG antibodies (Jackson ImmunoResearch Laboratories, Inc.). In this regard, as an experimental control of the galectin-3 binding, addition of 0.1 M of lactose (Sigma-Aldrich Co. LLC) for inhibiting the binding of galectin to sugar chains was performed. The measurement was performed by using a flow cytometer FACS CantoII (BD Biosciences), and the data were analyzed by using Kaluza (Beckman Coulter, Inc.) software.

As a result, the αβ T processed cells treated with 2DG had lower binding ability of galectin as compared with that in the case of the absence of 2DG (cont), and thus it was able to suggest that the binding ability of galectin-3 was reduced in a dose-dependent manner of 2DG (FIG. 6A).

### 8.2. Apoptosis induction by galectin-3

The αβ T processed cells were stimulated with 3 µM recombinant galectin-3 (BBI Solutions) under the condition of 0.1 M lactose (±) (Sigma-Aldrich Co. LLC). After washing the cells with PBS, the cells were stained with Propidium Iodide (PI, for detection of dead cells) and FITC-labeled Annexin V (for detection of apoptosis) by using Annexin V-FITC Apoptosis Detection Kit (NACALAI TESQUE, INC.). The stained cells were subjected to measurement by using a flow cytometer FACS CantoII (BD Biosciences), and the data were analyzed by using Kaluza (Beckman Coulter, Inc.) software. Fluorescein isothiocyanate (FITC)-Annexin V has a strong affinity for membrane phospholipids (phosphatidylserine, PS) existing inside the cell membrane, and therefore, when the PS is exposed to a surface of the cell membrane by apoptosis, Annexin V binds to the PS. In a case where there is no damage in the cell membrane, PI that binds to DNA is not incorporated into the cell. Apoptosis cells at a late stage were stained with both Annexin V and PI.

As a result, it was suggested that the apoptosis induction by galectin-3 is less likely to occur in the αβ T processed cells treated with 2DG (FIG. 6B). Therefore, the αβ T processed cells treated with 2DG can be expected to avoid the galectin-mediated immune-escape function of cancer cells even in the tumor environment, and can also be expected to exert a more anti-tumor effect in the tumor than the conventional αβ T processed cells.

### Example 9: In-vivo anti-tumor effect using immunodeficient mouse model

Immunodeficient mice (NOG mice, 6 to 8 weeks old) were purchased from CHARLES RIVER LABORATORIES JAPAN, INC., and were raised under the specific pathogen-free condition in accordance with the guidelines of the Animal Experiment Committee of Osaka University. All of the mice were injected via the tail vein on day 0 with 2 million human cancer cells (K562-Luc) in 200 µl of PBS. Subsequently, the αβ T processed cells treated with 2DG (2DG group in FIG. 7, seven NOG mice), or the αβ T processed cells not treated with 2DG (cont group in FIG. 7, six NOG mice) were injected intravenously once a week (day 0, day 7, day 14, and day 21 (shown by the symbol of ▲ in FIG. 7B)) at a dose of 20 million cells per mouse. The vehicle group (six NOG mice) was injected intravenously with 200 µl of PBS on day 0, day 7, day 14, and day 21.

The size of the tumor was measured on day 28 by In vivo Imaging System (IVIS) (Summit Pharmaceuticals International Corporation), and compared and investigated (FIG. 7A). In this regard, because one of the mice in the cont group died on day 27, the IVIS measurement of the cont group was performed on 5 mice. Furthermore, the survival time was also compared and investigated (FIG. 7B). In this regard, the changes in the survival rates of the cont group and vehicle group on day 28 and subsequent days showed the same trend, and the survival rates became 0 on day 32.

The mice injected with the αβ T processed cells treated with 2DG (2DG group) showed significantly smaller tumor size than the NOG mice treated with PBS (vehicle group) (FIG. 7A). Furthermore, the injection of the αβ T processed cells treated with 2DG significantly prolonged the survival of NOG mice, as compared with that of the mice by the treatment of the αβ T processed cells not treated with 2DG (cont) and of the mice treated with PBS (vehicle) (FIG. 7B). Therefore, the αβ T processed cells treated with 2DG promoted the in vivo anti-tumor cell effect (FIGS. 7A and 7B).

### INDUSTRIAL APPLICABILITY

By using the invention of the present application, cancer immunotherapy can be more specifically and selectively provided to the cancer that has expressed specific NKG2D ligands, than the direct administration of the drug. In addition, by altering the glycosylation on a cell surface of an αβ T processed cell, it can be expected to avoid the suppression of effector function in the tumor environment in a case where it is administered to the body.

## Claims

1. A method for preparation of an αβ T processed cell, comprising
in-vitro culturing an isolated peripheral blood mononuclear cell in the presence of
(1) IL-2,
(2) an anti-CD3 antibody, and
(3) 2-deoxy-d-glucose (2DG) or a derivative thereof.

2. The method according to claim 1, wherein
αβ T cells are contained at a proportion of around 74% to 90% and γδ T cells and NK cells are contained at a proportion of around 10% to 26%, in said αβ T processed cells.

3. The method according to claim 1, wherein
CD8 positive memory T cells are contained at a proportion of 78% or more in said αβ T processed cells.

4. The method according to claim 1, wherein
IL-2R positive αβ T cells are contained at a proportion of 25% or more, and/or perforin positive αβ T cells are contained at a proportion of 15% or more and/or granzyme positive αβ T cells are contained at a proportion of 20% or more, in said αβ T processed cells.

5. The method according to any one of claims 1 to 3, wherein
a derivative or analog of 2DG is selected from the group consisting of 2-deoxymannose-6-phosphate, 2-deoxymannose-1-phosphate and GDP-2-deoxymannose, 2-fluoro-2-deoxyglucose, 2-fluorodeoxyglucose, 2-fluoro-deoxyglucose-6-phosphate, 2-fluoro-deoxyglucose-1-phosphate, 2-fluoro-deoxymannose, 2-fluoro-deoxymannose-6-phosphate, 2-fluoro-deoxymannose-1-phosphate, or a sugar having a six-membered ring such as galactose or mannose and a derivative thereof, and a physiologically acceptable salt or a solvate thereof.

6. A pharmaceutical composition comprising αβ T processed cells stimulated with 2-deoxy-d-glucose (2DG) or a derivative thereof, for the treatment or prevention of cancer.

7. The pharmaceutical composition according to claim 6, wherein
said cancer expresses a NKG2D ligand.

8. The pharmaceutical composition according to claim 7, wherein
the NKG2D ligand is selected from the group consisting of MICA, MICB, RAET1ε, RAET1α, RAET1β, RAET1γ, RAET1δ, Mult1, H60a, H60b, H60c, and ULBPs 1 to 6.

9. The pharmaceutical composition according to claim 7, wherein
the cancer expressing the NKG2D ligand is selected from the group consisting of osteosarcoma, breast cancer, cervical cancer, ovarian cancer, endometrial cancer, bladder cancer, lung cancer, pancreas cancer, colon cancer, prostate cancer, leukemia, acute lymphocytic leukemia, chronic lymphocytic leukemia, B-cell lymphoma, Burkitt's lymphoma, multiple myeloma, Hodgkin's lymphoma, non-Hodgkin's lymphoma, myeloid leukemia, acute myeloid leukemia (AML), chronic myeloid leukemia, thyroid cancer, follicular thyroid cancer, myelodysplastic syndrome (MDS), fibrosarcoma, rhabdomyosarcoma, melanoma, uveal melanoma, teratocarcinoma, neuroblastoma, glioma, glioblastoma, keratoacanthoma, kidney cancer, anaplastic large-cell lymphoma, esophageal squamous cell carcinoma, hepatocellular carcinoma, follicular dendritic cell carcinoma, intestinal cancer, muscle invasive cancer, testicular cancer, epidermal cancer, spleen cancer, head and neck cancer, gastric cancer, liver cancer, bone cancer, brain cancer, retinal cancer, biliary tract cancer, small intestinal cancer, salivary gland cancer, uterine cancer, testicular cancer, connective tissue cancer, benign prostatic hyperplasia, myelodysplasia, waldenstrom's macroglobulinemia, nasopharyngeal cancer, neuroendocrine carcinoma, mesothelioma, angiosarcoma, Kaposi's sarcoma, carcinoid, esophageal gastric cancer, fallopian tube cancer, peritoneal cancer, serous papillary Muller tube cancer, malignant ascites, gastrointestinal stromal tumor (GIST), and Li-Fraumeni syndrome.

10. A pharmaceutical composition comprising αβ T processed cells stimulated with 2-deoxy-d-glucose (2DG) or a derivative thereof, for the treatment or prevention of infection with a bacterium or a virus.

11. The pharmaceutical composition according to claim 10, wherein
the bacterium is selected from a Gram-negative bacterium or a Gram-positive bacterium.

12. The pharmaceutical composition according to claim 10, wherein
the virus is selected from the group consisting of cytomegalovirus, influenza virus, Epstein-Barr virus (EBV), adenovirus, hepatitis B virus, hepatitis C virus, human papillomavirus, human T-lymphotropic virus (HTLV), and human immunodeficiency virus (HIV).

13. Use of the αβ T processed cells prepared by the method according to any one of claims 1 to 5 in production of a product for regenerative medicine or the like.

14. The use according to claim 13, wherein
the product for regenerative medicine or the like is a product for regenerative medicine or the like for the treatment or prevention of cancer.

15. The use according to claim 13, wherein
the product for regenerative medicine or the like is a product for regenerative medicine or the like for the treatment or prevention of infection with a bacterium or a virus.
